# EUROPEAN PATENT SPECIFICATION

(11) **EP 1 061 957 B1**
(45) Date of publication and mention of the grant of the patent: **30.10.2002**
(21) Application number: 99910336.9
(22) Date of filing: 09.03.1999
(51) Int. Cl.: A61K 49/00, C07C 229/28, C07C 229/36, C07C 233/83, C07D 209/20, C07C 229/22

(54) **MANGANESE (II) CHELATES WITH HIGH RELAXIVITY IN SERUM**
MANGAN (II) CHELATE MIT HOHER IN-SERUM RELAXIVITÄT
CHELATES DE MANGANESE (II) PRESENTANT UN POUVOIR DE RELAXATION ELEVE DANS LE SERUM

(30) Priority: 10.03.1998 IT MI980477
(43) Date of publication of application: 27.12.2000
(73) Proprietor: BRACCO S.p.A., 20134 Milano (IT); BRACCO IMAGING S.p.A., 20134 Milano (IT)
(72) Inventor: BROCCHETTA, Marino, I-20134 Milano (IT); CALABI, Luisella, I-20134 Milano (IT); FEDELI, Franco, I-20134 Milano (IT); MANFREDI, Giuseppe, I-20134 Milano (IT); PALEARI, Lino, I-20134 Milano (IT); UGGERI, Fulvio, I-20134 Milano (IT)
(74) Representative: Minoja, Fabrizio, Dr.
(86) International application number: EP9901500
(87) International publication number: WO99045968

(56) References cited:
- EP-A- 0 230 893
- EP-A- 0 279 307
- EP-A- 0 822 180
- WO-A-98/05625
- US-A- 4 980 148
- KEANA, JOHN F. W. ET AL: "Chelating ligands functionalized for facile attachment to biomolecules. A convenient route to 4-isothiocyanatobenzyl derivatives of diethylenetriaminepentaacetic acid and ethylenediaminetetraacetic acid" J. ORG. CHEM. (1990), 55(9), 2868-71 CODEN: JOCEAH;ISSN: 0022-3263, XP002104514
- WESTERBERG, DAVID A. ET AL: "Synthesis of novel bifunctional chelators and their use in preparing monoclonal antibody conjugates for tumor targeting" J. MED. CHEM. (1989), 32(1), 236-43 CODEN: JMCMAR;ISSN: 0022-2623, XP002104515

## Description

The present invention relates to the field of the diagnostic technique known as Magnetic Resonance Imaging (M.R.I.), a diagnostic procedure used in medical practice to detect anomalies in organs or tissues of the human or animal body (see: Rocklage S.M., Watson A.D. and Carvlin M.J., Magnetic Resonance Imaging, Chap. 14, Vol.1, Second Ed., 1992; Stark D.D. and Bradley W.G. Eds.). Particularly, the invention relates to manganese chelated complexes of ethylenediaminotetraacetic acid derivatives, the physiologically compatible salts thereof and the use of these compounds as M.R.I. contrast agents.

The potential usefulness of the Mn²⁺ ion as an M.R.I. contrast agent has been taken in consideration since 1978 for myocardium imaging. In Magnetic Resonance in Medicine 1988, 8, 293-313, the longitudinal proton relaxation times in rabbit tissues after intravenous administration of MnCl₂ and of Mn-PDTA (1,3-propylenediamino-N,N',N'',N'''-tetraacetate) have been evaluated. Manganese complexes of porphyrins and derivatives thereof (for example Mn(III)-meso-tetra(4-solfonatophenyl)porphyrin, Mn-TPPS₄) were proposed as contrast agents specific for tumors (Investigative Radiology 1995, ***30(10)***, 611-620).

In a recent study, M.R.I. contrast agents were evaluated consisting of Mn-EDTA lipohilic derivatives (for example, manganese-EDTA-bis(hydroxypropyldecylamine), Mn-EDTA-DDP) bound to the membranes of small unilamellar liposomes ("memsomes"), which are potentially valuable for hepatic and cardiac perfusion imaging (Journal of Liposome Research 1994, *4(2)*, 811-834). The same compounds are object of International Patent Application WO 92/21017 and of US 5,312,617, which disclose M.R.I. contrast agents based on manganese chelates (II) consisting of EDTA bis-amides where the amide nitrogens are substituted with long chain alkyl residues (C₇-C₃₀). These chelates per se or, more preferably, in combination with lipids or liposomes, are reportedly particularly useful for imaging of liver and as blood-pool agents.

M.R.I. contrast agents comprising manganese chelated complexes are also described in US patents 4,980,148 and 5,246,696. Said complexes, in which the ligand is an alkylenediaminotetraacetic acid in which the alkylene chain is interrupted by one or more substituents selected from O, S, CHOH, CHSH, are stated to be particularly useful for imaging of liver, kidneys, pancreas and gastrointestinal tract.

Recently, Teslascan^{(R)} (manganese complex of N,N'-1,2-ethanediylbis[N-[[3-hydroxy-2-methyl-5-[(phosphonooxy)methyl]-4-pyridinyl]-methyl]glycine salified with Na⁺ (1:3), mangafodipir trisodium, was marketed in Europe and in the U.S.A. for use in M.R.I. of the liver. This compound was considered useful for M.R.I. diagnosis of pancreas adenocarcinoma and pancreatitis (Investigative Radiology 1995, *30(10)*, 611-620).

Contrary to the Gd(III) ion (at present the M.R.I. contrast agent of choice) and, more generally, to the lanthanides ions, manganese is an essential oligoelement present in all mammal cells: this is undoubtedly an advantage as for the tolerability of the corresponding complexes. However, the manganese ion can cause toxicity when released from the corresponding chelates *in vivo;* this mainly applies when the manganese ion is administered intravascularly, as in this case the natural regulation of its adsorption by the intestine fails. It is therefore necessary for it to be administered as stable complex, in order to prevent any toxicity due to the free metal and to improve its elimination.

Some compounds of the above cited literature, for example Mn-DPDP, show some instability *in vivo*: a recent biodistribution study proved that this complex dissociates, releasing manganese which accumulates in liver, pancreas and kidneys, whereas the undissociated chelate is removed through glomerular filtration. The M.R.I, properties of Mn-DPDP would therefore be ascribable mainly to the manganese ion released by the complex, which accumulates in liver and pancreas (Investigative Radiology 1994, *29(2)*, S249-S250). Another recent study gave evidence of the dissociation of Mn-DPDP in liver homogenate, in the presence of calcium and magnesium ions (MRM 1996, 35, 14-19).

EP230893 discloses compounds suitable for NMR imaging based on a ethylenediaminotetraacetic acid nucleus differently substituted on the acetyl moieties, which form complexes with divalent ions such as manganese (2+). The complexes can be used as contrast agents in nuclear spin tomography and are particularly suited for the imaging of the gastro-intestinal tract.

Besides the problem of the *in vivo* stability, a further problem of manganese chelated complexes is that of relaxivity, that is usually lower than the better studied and developed gadolinium contrast agents.

At present, in the field of M.R.I. contrast agents, there are no valuable manganese contrast agents having good stability as well as high relaxivity.

The compounds of the present invention solve this problem in the field of M.R.I, diagnostic imaging. A class of compounds selected from EP 230 893, more precisely a class of Mn(II) chelates, has, in fact, shown some unexpected properties which make them valuable for use in this field.

The present invention relates to stable manganese chelated complexes in which said ion is in the oxidation state +2 (Mn(II)).

More particularly, the invention relates to Mn(II) chelates of compounds of formula (I): 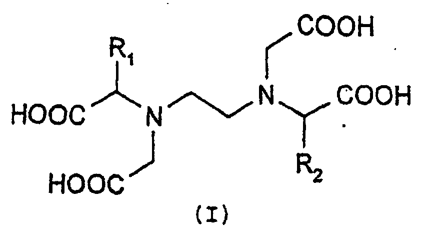 wherein:
R₁, R₂ which are the same or different, are a -(CH₂)ₘ-O-(CH₂)ₙ-R₃ group, wherein R₃ is an aryl residue, m is an integer 1 to 5 and n is an integer 1 to 4,
as well as the optically active forms of the chelates thereof with Mn(II) and the salts thereof with physiologically compatible organic bases selected from primary, secondary, tertiary amines or basic amino acids, or with inorganic bases whose cations are sodium, potassium, magnesium, calcium, or mixtures thereof.

Suitable substances for salifying the chelates of the invention are, for example:
- cations of inorganic bases such as alkali or alkaline-earth metals ions selected from sodium, potassium, magnesium, calcium, or mixtures thereof;
- cations of physiologically compatible organic bases selected from primary, secondary and tertiary amines such as ethanolamine, diethanolamine, morpholine, glucamine, N-methylglucamine, N,N-dimethylglucamine;
- cations of amino acids such as those of lysine, arginine or ornithine.

Particularly preferred are N-methylglucamine salts.

In the compounds of formula (I) particularly preferred meanings for R₁ and R₂ are the following: 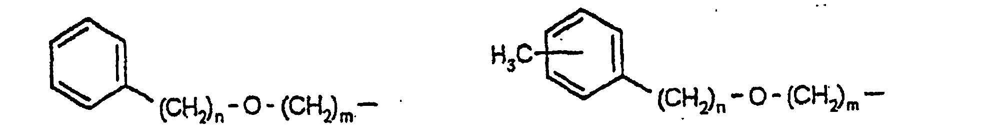 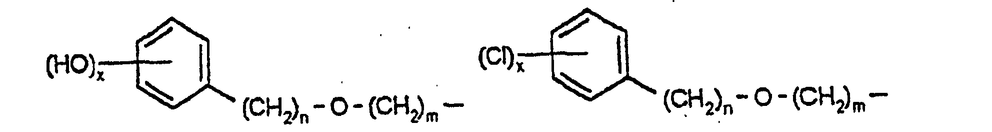 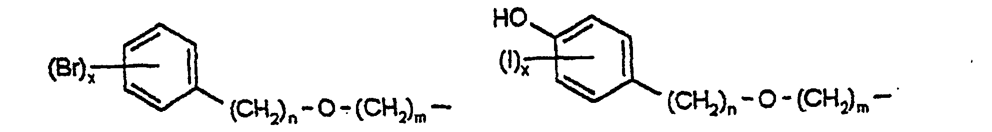 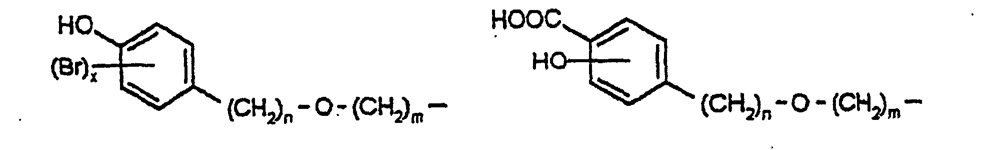 wherein: x = 1,2.

Particularly preferred compounds within formula (I), are those of formula (II): 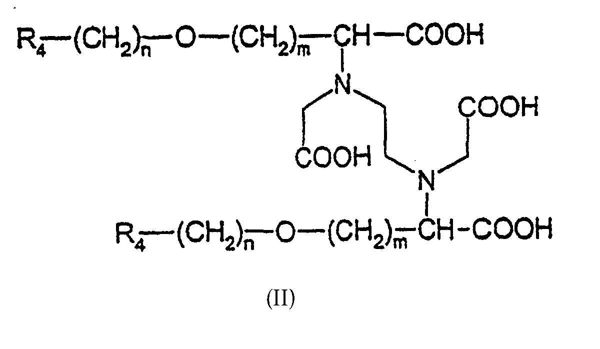 wherein the R₄ groups, independently of one another, are phenyl, unsubstituted or substituted with one or more R₅ groups, which are the same or different, wherein R₅ is halogen, -OH, -NH₂, -COOH, -CONH₂, -SO₃H, -SO₃NH₂, or a C₁-C₄ alkyl chain.

In compounds of formula (II) , preferred meanings for R₄ are the following ones: 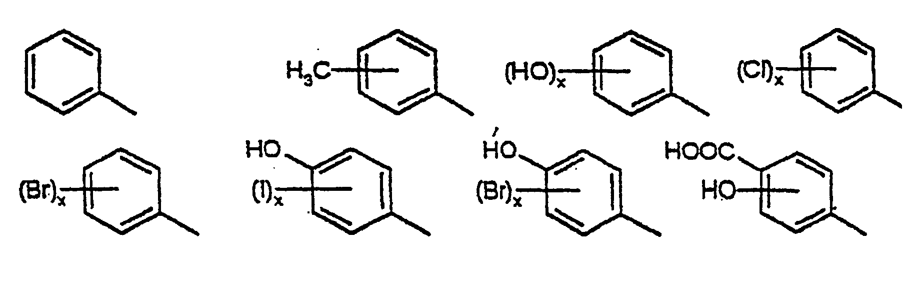 wherein: x = 1,2.

Within formula (I), particularly preferred compounds are those of formula (III) 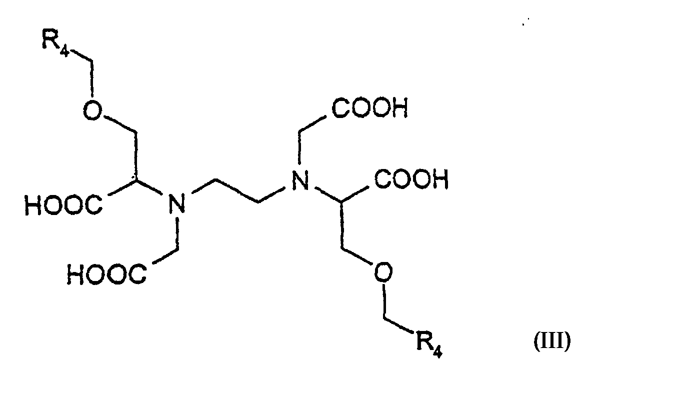 wherein the R₄ groups, which are the same or different, have the same meanings as defined for compounds (II).

In compounds of formula (III), preferred meanings for R₄ are as follows:

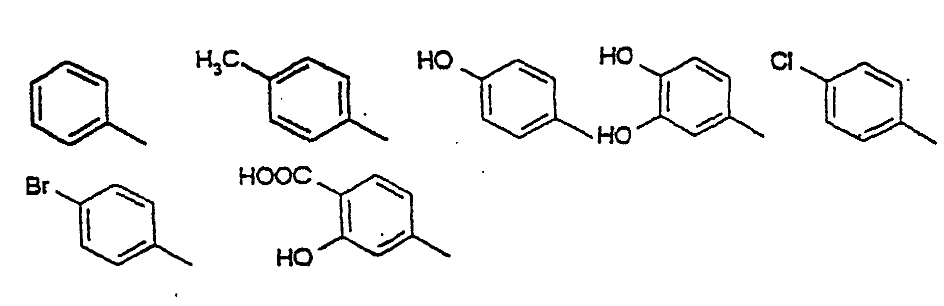

Particularly preferred is the unsubstituted phenyl residue.

Among the possible manganese chelates included within formula (I), particularly preferred is the following:

### COMPOUND 1 (EXAMPLE 1)

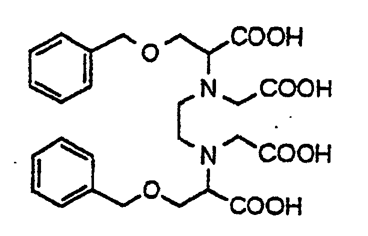

The present invention also relates to the use of the Mn²⁺ complex chelates of the chelating compounds of formulae (I) to (III) and to the salts thereof, for the preparation of pharmaceutical compositions for diagnostic use, as well as the formulations themselves.

The compounds of the invention can conveniently be used as they are or they can be conjugated with macromolecules or incorporated in structures which carry them to specific body regions.

The compounds of the invention can be formulated with additives conventionally used for the pharmaceutical or veterinary formulations, for example stabilizers, antioxidants, osmolality and pH adjusters, buffers and the like.

For the parenteral administration, they are preferably formulated as sterile aqueous solutions or suspensions, whose pH can range from 6.0 to 8.5.

Said formulations can also be lyophilized and supplied as such for reconstitution before use. For the gastrointestinal use or for the injection into body cavities, these agents can be formulated as a solution or suspension containing suitable additives in order to, for example, control viscosity.

For the oral administration they can be formulated according to preparation methods routinely used in the pharmaceutical technique, optionally also as coated formulations to gain extra protection from the acid pH of stomach, thereby inhibiting the release of the chelated metal ion, which usually occurs at the typical pH values of gastric juices.

Other excipients, such as sweeteners and/or flavours, can also be added according to known techniques of pharmaceutical technique.

The solutions or suspensions of the compounds of this invention can also be formulated as aerosol for use in aerosol-bronchography.

Contrary to the manganese chelated complexes of the prior art cited above, the compounds of the invention have shown characteristics such as good tolerability, good stability both *in vitro* and *in vivo* and surprisingly high r₁ and, particularly, r₂ relaxivities in human serum. The relevant data are summarized in Tables 1, 2 and 3. These characteristics make the compounds of the invention particularly interesting as M.R.I. contrast agents of general use.

The chelates of the invention have also shown a good applicability in the imaging of the liver (Table 4) and of the biliary tracts, which organs are reached by said chelates without significant losses of the chelated metal, thus confirming their characteristics of stability. This is a further aspect of the present invention.

Contrary to the manganese chelated complexes of the prior art, which, as mentioned above, are generally considered particularly useful in the imaging of the liver, the pancreas and the gastrointestinal tract, the chelates of the present invention, thanks to their surprising relaxivity values in human serum, can advantageously improve the images obtainable using said compounds in formulations specific for the imaging of the cardiocirculatory system. This is a further aspect of the present invention.

Furthermore, the chelated complexes of the present invention are characterized in having particularly high transversal relaxivity values (r₂) in human serum. This characteristic makes them unexpectedly unique compared with the general teaching of EP 230 893, that mainly focuses on the effects of the compounds on relaxation times T₁. This makes them most suitable, and this is a further aspect of the present invention, for use in M.R.I. diagnostic imaging in recording images of organs or tissues, wherein said images are acquired by combinations of T₁/T₂ sequences or by T₂ weighed sequences.

According to the present invention, said chelated complexes proved to be particularly suitable also in the preparation of pharmaceutical formulations for M.R.I. diagnostic imaging at low fields, i.e. fields ranging from 0.1 to 0.5 Tesla or, when measuring the intensity of the applied magnetic field in Hertz, fields below 20 MHz (An introduction to Magnetic Resonance in Medicine, Ed. Peter A. Rinck). The compounds of the invention, thanks to their excellent relaxivity shown even at such low fields, can advantageously be used with apparatuses such as Artroscan (arthrographs which make use of the Magnetic resonance), open apparatuses, apparatuses intended for diagnostic of single regions of the human body (e.g. the shoulder), and generally apparatuses less expensive, easier to handle and increasingly diffused.

Furthermore, and this is a further aspect of the present invention, the considerable increase in relaxivity in human serum shown by the compounds of the invention allows the use thereof in diagnostic formulations requiring a low dosage of the contrast agent. This can be attained:
a) by administering a reduced volume of contrastographic solution;
b) by administering more diluted contrastographic compositions (low-dosage compositions).

Said compositions provide images of good quality, while assuring a significant improvement from the toxicologic point of view.

The contrastographic formulations containing the chelated complexes of the invention can therefore be formulated with concentrations of contrast agent ranging from 0.001 to 1.0 mmol/mL, preferably from 0.01 to 0.5 mmol/mL; in particular, concentrations lower than 0.25 mmol/mL for the low-dosage formulations.

Said formulations can be administered to the patient in doses ranging from 0.001 to 0.1 mmol/kg, preferably 0.1 mmol/kg; in particular, doses ranging from 5 to 50 µmol/kg when administered in low dosages.

Among the possible synthetic routes which can be used for the preparation of the compounds of the invention, some general schemes are reported in the following.

When R₁ is the same as R₂, the procedure summarized in the following Scheme 1 will be followed: 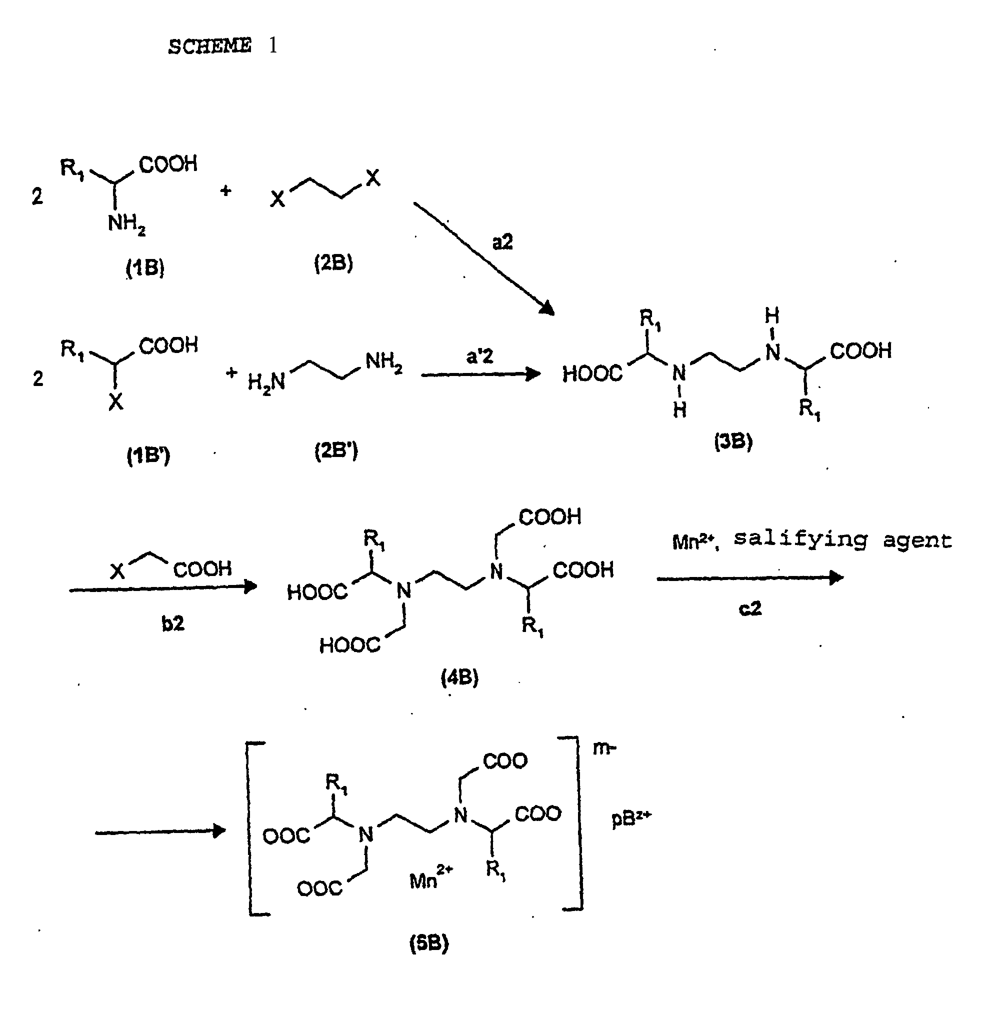 wherein:
- R₁: has the same meanings as in formula (I);
- X =: halogen (Cl, Br, I);
- m =: number of the total charges of the chelated complex;
- B =: substance suitable for salifying the chelated complex (for example Na⁺, K⁺, Mg⁺⁺, Ca⁺⁺ or mixtures thereof, meglumine, etc.);
- z =: number of the charges of B;
- p: is such an integer that the product: p · z = m.

In step (a2) a suitable α-amino acid (1B) is reacted with a 1,2-dihaloethane, such as 1,2-dibromoethane (2B) in a suitable solvent (e.g. water/ethanol, water/methanol mixtures), keeping pH at about 9 with a suitable buffer (preferably 2M borate buffer) at a temperature which can range from 60 to 90°C. Alternatively, (step a'2), an α-haloacid (1B') can be reacted with 1,2-diaminoethane (2B') in aqueous solution at 20 to 60°C. The resulting intermediate (3B) is carboxymethylated in step (b2) with an α-haloacetic acid (e.g. bromoacetic acid) at basic pH (about 10) and at a temperature from 40 to 70°C, to give the free ligand (4B). This is reacted in step (c2) with the stoichiometric amount of manganese, according to the general procedure already described for scheme 1, to obtain the desired chelated complex (5B).

Using a different stoichiometry, asymmetric ligands can be prepared, wherein R₁ and R₂ are both different from hydrogen and different from each other, as represented in the following Scheme 2: 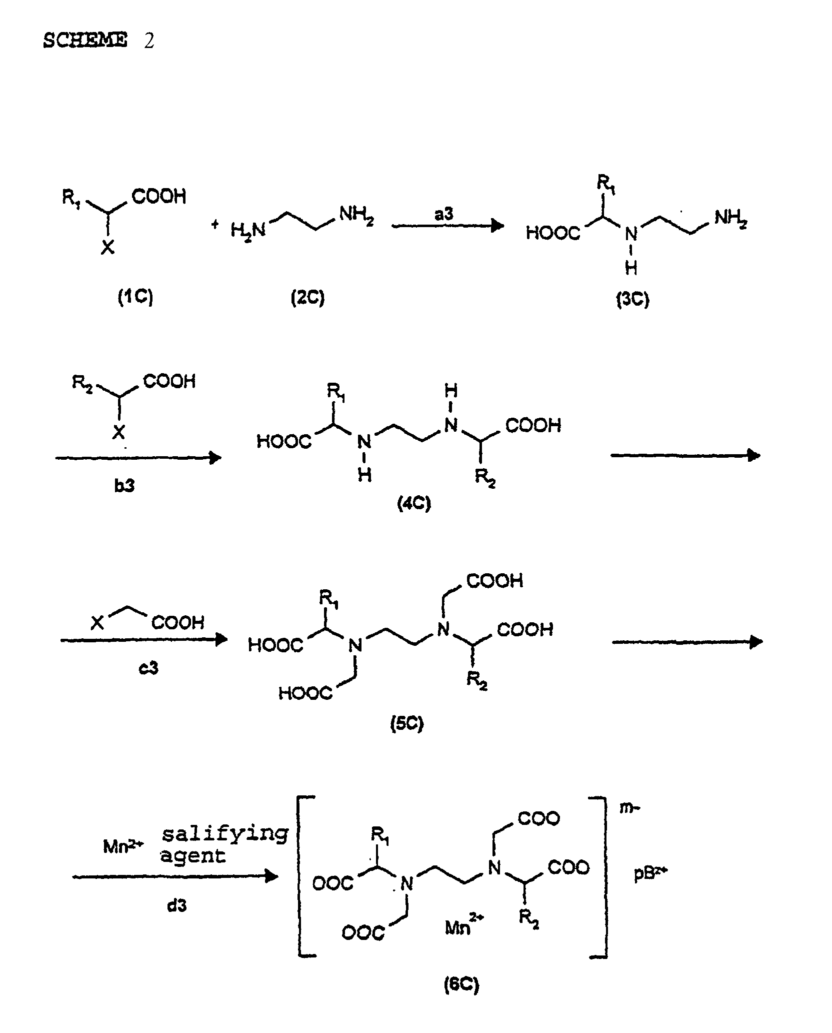 wherein:
- R₁, R₂: have the same meanings as in formula (I);
- X =: halogen (Cl, Br, I);
- m =: number of the total charges of the chelated complex;
- B =: substance suitable for salifying the chelated complex (for example Na⁺, K⁺, Mg⁺⁺, Ca⁺⁺ or mixtures thereof, meglumine, etc.);
- z =: number of the charges of B;
- p: is such an integer that the product: p · z = m.

An alternative process which provides disubstituted compounds is represented by the following Schemes 3A and 3B: 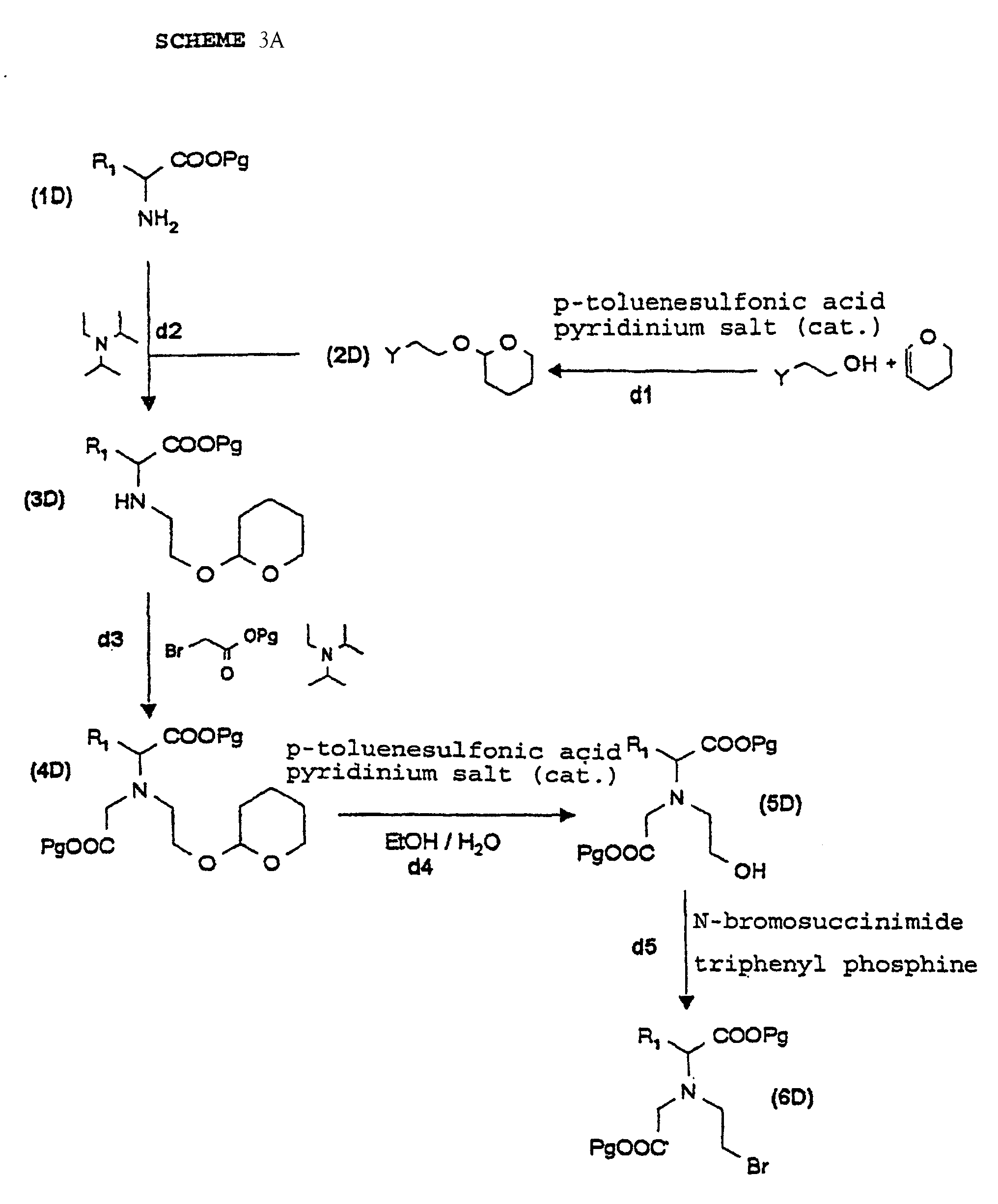 wherein:
R₁ has the same meanings as in formula (I);
Pg = protective group (e.g. t-butyl);
Y = Cl, Br or other leaving groups (I, -OMs, -OTf, -OTs).

Step (d1) comprises the protection of the alcoholic fraction of a 2-haloethanol (preferably 2-bromoethanol) with dihydropyran, to give intermediate (2D). The alcohol-protecting group can be, for example, benzyl and trityl. The reaction takes place in an organic solvent such as CH₂Cl₂, CHCl₃, CH₂ClCH₂Cl, in the presence of 4-toluenesulfonic acid pyridinium salt or of another acid catalyst. In intermediate (2D), the leaving group is preferably Br.

In step (d2) the ester (e.g. t-butyl ester) of a natural or synthetic α-amino acid (1D), either in the racemic or optically active form, is reacted with the intermediate (2D) in the presence of a base, such as diisopropylethylamine, in a solvent such as CH₃CN, DMF or a chlorinated solvent, to give intermediate (3D).

The latter is reacted, in step (d3), with a bromoacetic acid ester (e.g. t-butyl-bromoacetate) in the presence of a base, such as diisopropylethylamine, to give intermediate (4D) which is reacted, in the subsequent step (d4), with 4-toluenesulfonic acid pyridinium salt or another acid catalyst, in an ethanol/water mixture, at 20-60°C, to give intermediate (5D).

In step (d5), intermediate (5D) is brominated with N-bromosuccinimide in the presence of triphenylphosphine, to give intermediate (6D), which is reacted, in step (d6) with the ester (e.g. t-butyl ester) of an α-amino acid (7D), in a suitable solvent (e.g. CH₃CN/buffer phosphate pH 8), to give intermediate (8D). This is then reacted with a bromoacetic acid ester (e.g. t-butyl-bromoacetate) in the presence of a base, such as diisopropylethylamine, to give tetraester (9D). The latter is deprotected in step (d8) with known methods (e.g. hydrolysis with CF₃COOH or (CH₃)₃SiI, when the protective group is *t*-butyl) to give the free ligand (10D), which is subsequently complexed and salified according to the general procedure already described in the above schemes. 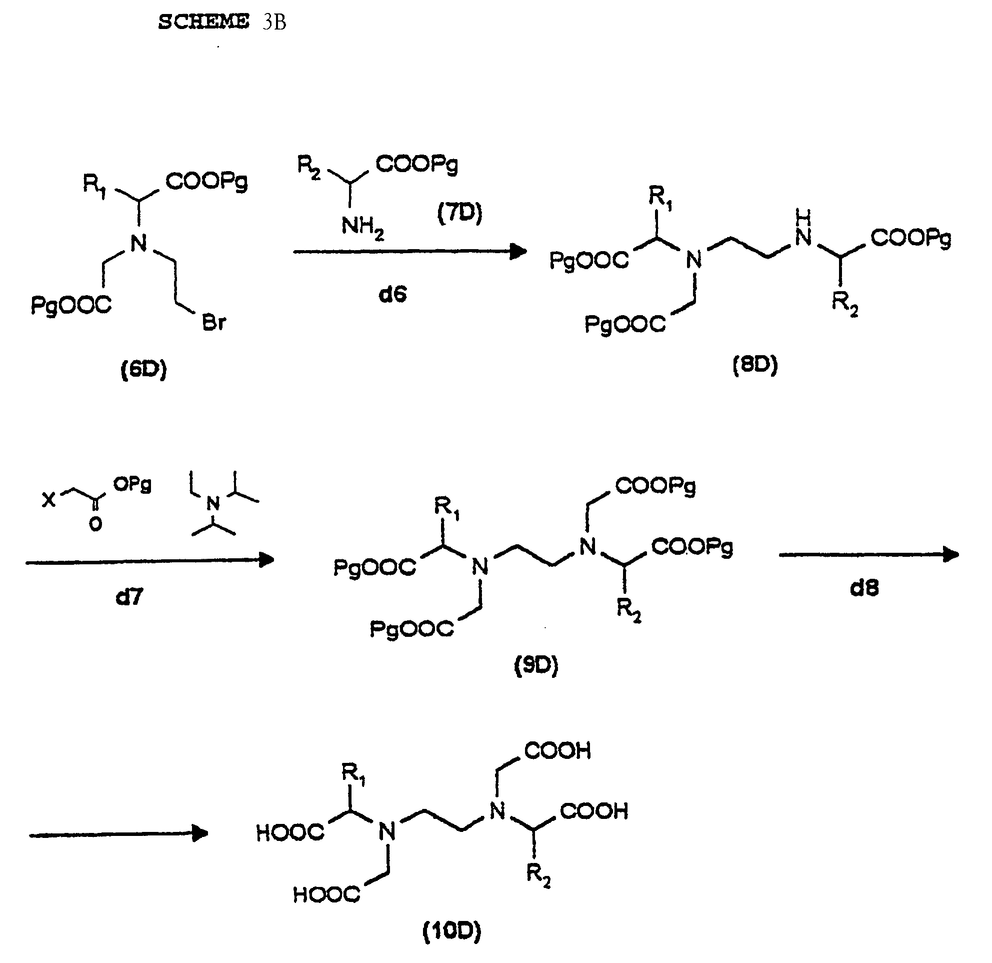 wherein:
- R₁, R₂: have the same meanings as in formula (I);
- Pg =: protective group (e.g. t-butyl);
- X =: halogen (Cl, Br, I).

As for, in particular, the compounds of formula (III), when the R₄ groups are different from each other, the synthetic routes of Schemes 2 and 3 above will be followed; when the R₄ groups are the same, the preferred synthetic scheme is the following: 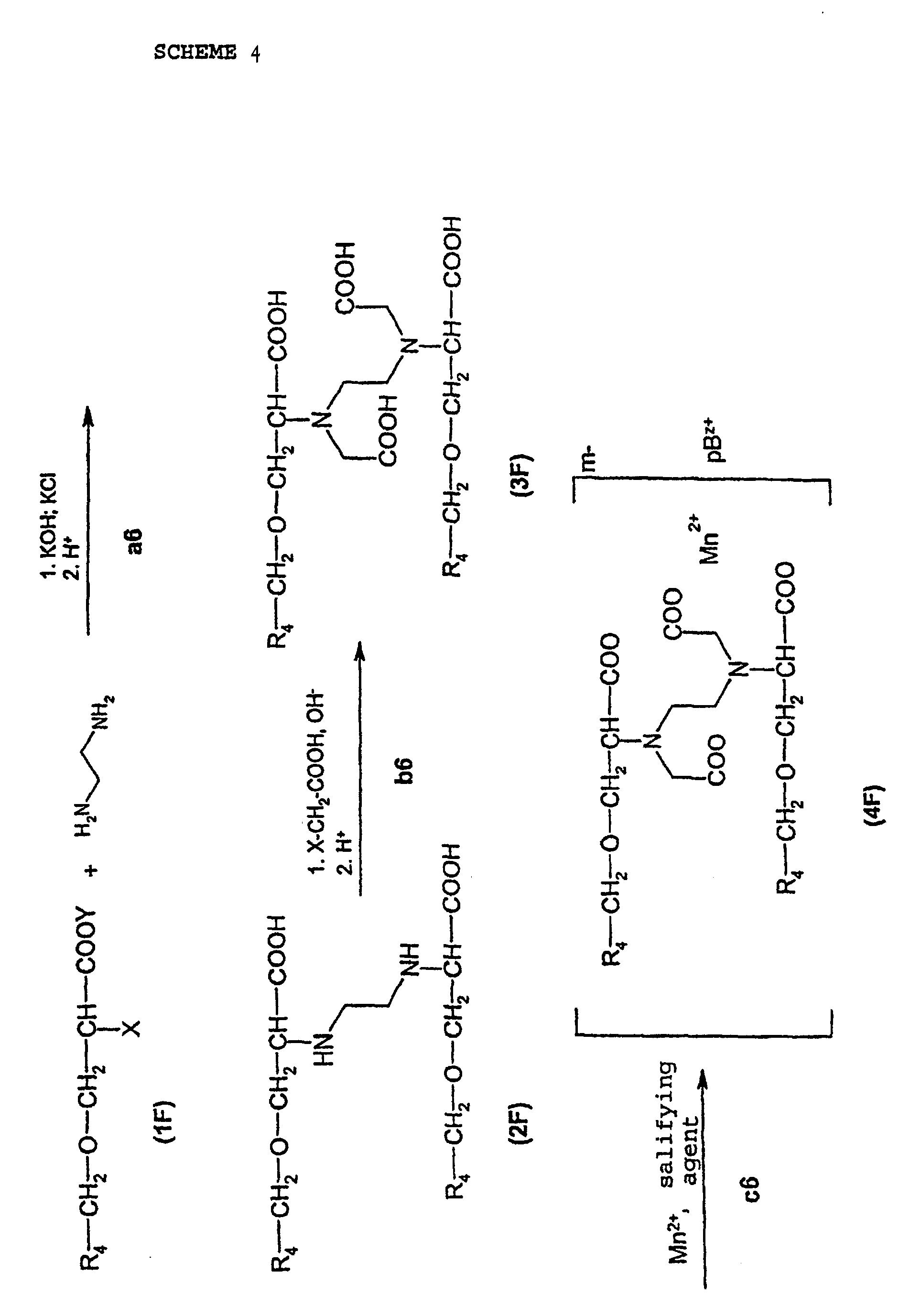 wherein:
- R₄: has the same meanings as in formula (III);
- X =: halogen (Cl, Br, I);
- Y: is a cation of an alkali metal selected from Na, K;
- m =: number of the total charges of the chelated complex;
- B =: substance suitable for salifying the chelated complex (for example Na⁺, K⁺, Mg⁺⁺, Ca⁺⁺ or mixtures thereof, meglumine, etc.);
- z =: number of the charges of B;
- p: is such an integer that the product: p · z = m.

In step (a6), the salt (preferably the potassium salt) of a 3-benzyloxy-2-halopropionic acid (1F), optionally substituted at the benzene ring, wherein the halogen at the 2- position is selected from Cl, Br, I, preferably Cl, is reacted in aqueous solution at basic pH (preferably pH 12) by addition of inorganic bases (preferably KOH) with 1,2-diaminoethane in the presence of KCl at a temperature from 50 to 80°C, preferably 65°C. The subsequent acidification yields intermediate (2F) which, in step (b6), is carboxymethylated with a 2-haloacetic acid (preferably 2-bromoacetic acid) at basic pH (about 10) and at 40 to 70°C, preferably 50°C. The subsequent acidification gives the free ligand (3F) that is reacted, in the subsequent step, with the stoichiometric amount of manganese, according to the general procedure already described in the above schemes, to obtain the desired chelated complex (4F).

An innovative aspect of this process compared with what reported, for example, in EP 230893, and particularly applicable to the product described in the subsequent Example 1, consists in reacting, in step (a6), the potassium salt of acid (1E), instead of the corresponding acid, with ethylenediamine in the presence of KCl. The use of the latter is the key to obtain the product in good yields. A further aspect is represented by temperature, which should be as near as possible to 65°C: lower temperatures decrease the reaction yield and temperatures higher then 65°C involve undesired side-reactions. The process described above is a further aspect of the present invention.

The following examples illustrate the best experimental conditions to prepare the compounds of the invention.

### EXAMPLE 1

Manganese complex(II) of *N,N'*-1,2-ethanediylbis[*N*-(carboxymethyl)-*O*-(phenylmethyl) -D,L-serine] salified with 1-deoxy-1-(methyl amino) -D-glucitol (1:2)

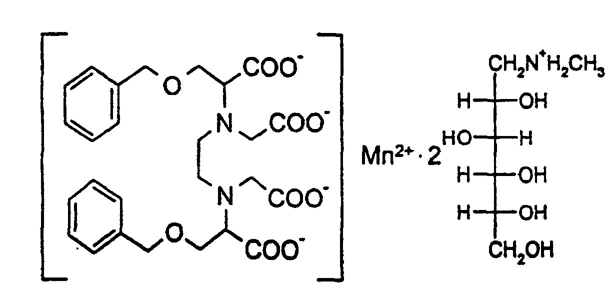

### A) N,N'-1,2-ethanediylbis[O-(phenylmethyl)-D,L-serine]

A solution of 3-benzyloxy-2-chloropropionic acid potassium salt (25.27 g; 0.1 mol), ethylenediamine (1.98 g; 0.033 mol) and KCl (12.37 g; 0.166 mol) in H₂O (66 mL), was heated at 65°C for 48 hours, adding 8 N KOH (12.1 mL; 0.097 mol) by means of a pH-stat to keep pH 12. The reaction mixture was cooled at r.t., diluted with H₂O (120 mL) and acidified (pH 6) with 1 N HCl (85 mL) under strong stirring. The precipitate was filtered, washed with H₂O and dried (P₂O₅, 50°C, 2 kPa) to give the desired product (8.3 g; 0.02 mol). Yield 61%.
- m.p.:: 213°C (dec.)
- Acidic titer (0.1 N NaOH):: 96%
- TLC:: Rf 0.75
Silica gel plates 60 F₂₅₄ (KGaA Merck art. 5715)
Eluent = CHCl₃/AcOH/H₂O 5:5:1
Detection: 1% KMnO₄ in 1 N NaOH
- HPLC :: 97% (% area)
- K.F. :: 0.56 %

The ¹H-NMR, ¹³C-NMR, IR and MS spectra are consistent with the indicated structure.

| Elementary analysis (%): | | | |
|---|---|---|---|
| | C | H | N |
| Calculated | 63.45 | 6.78 | 6.73 |
| Found | 63.53 | 6.81 | 6.75 |
| | | | on the anhydrous |

### B) N,N'-1,2-ethanediylbis[N-(carboxymethyl)-O-(phenylmethyl)-D,L-serine]

A suspension of the product from the previous step (216 g; 0.518 mol) and bromoacetic acid (217 g; 1,56 mol) in H₂O (1.8 L), was added with 10 N NaOH to pH 10. The mixture was heated at 50°C for 22 hours, keeping pH at 10 by addition of 10 N NaOH by means of a pH-stat. After cooling at r.t., the reaction mixture was diluted with H₂O (0.5 L) and acidified (pH 1.5) with 6 N HCl (0.4 L) to obtain a precipitate which was separated by decantation and then suspended in H₂O (3 L). After 18 hours the precipitate was again separated by decantation, powdered in a mortar, filtered, washed with H₂O and dissolved in 1 N NaOH (1,85 L). After dilution with H₂O (2.45 L), the solution was acidified (pH 3) with 2 N HCl, with strong stirring, filtered and then acidified to pH 1.5 with 2 N HCl. After 24 hours the precipitate was separated by filtration, powdered in a mortar, washed with H₂O (0.5 L) and dried (P₂O₅; 50°C; 2 kPa). The resulting solid was suspended in *n*-butanol saturated with water (3 L) and stirred at r.t. for 18 hours. The resulting suspension was filtered and the solid residue was washed with n-butanol saturated with water (1.5 L) and dried (P₂O₅; 60°C; 2 kPa), to give the desired product (178.4 g; 0.335 mol). Yield 65%.
- m.p.:: 158-159°C
- Acidic titer (0.1 N NaOH):: 99.4%
- Complexometric titer (0.1 N ZnSO₄):: 98.5%
- TLC :: Rf 0.53
Silica gel plates 60 F₂₅₄ (KGaA Merck art. 5715)
Eluent = CHCl₃/AcOH/H₂O 5:5:1
Detection: 1% KMnO₄ in 1 N NaOH
- HPLC :: 97.3% (% area)
- K.F. :: 3.10%

The ¹H-NMR, ¹³C-NMR, IR and MS spectra are consistent with the indicated structure.

| Elementary analysis (%): | | | |
|---|---|---|---|
| | C | H | N |
| Calculated | 58.64 | 6.06 | 5.26 |
| Found | 58.47 | 6.06 | 5.30 |
| | | | on the anhydrous |

### C) Manganese complex(II) of N,N'-1,2-ethanediylbis[N-(carboxymethyl)-O-(phenylmethyl)-D,L-serine] salified with 1-deoxy-1-(methylamino)-D-glucitol (1:2).

A solution of MnCl₂ · 2 H₂O (11.33 g; 0.07 mol) in H₂O (50 mL) was dropped in 2 hours into a solution of the free ligand from the previous step (37.28 g; 0.07 mol) in H₂O (300 mL), neutralized (pH 7) adding 1 N meglumine (273 mL; 0.273 mol) by means of a pH-stat. The reaction mixture was filtered through a Millipore HA 0.45 µm filter, diluted with H₂O (2 L) and nanofiltered. The retentate was evaporated under vacuum (2 kPa) to obtain a solid residue which was dried (P₂O₅, 50°C, 2 kPa) to give the desired product (52.9 g; 0.0542 mol). Yield 77%.
- m.p.:: 95-100°C
- Free ligand (HPLC):: < 0.01%
- HPLC:: 99.7% (% area)
- K.F.:: 4.71%

The IR and MS spectra are consistent with the indicated structure.

| Elementary analysis (%): | | | | |
|---|---|---|---|---|
| | C | H | Mn | N |
| Calculated | 49.23 | 6.61 | 5.63 | 5.74 |
| Found | 49.18 | 6.56 | 5.13 | 5.64 |
| | | | on the anhydrous | |

The relaxivity of the compounds of the invention was measured both in saline solution and in human serum, reconstituted from Seronorm^{(R)} Human (Nycomed Pharma), lyophilized human serum. The obtained data are summarised in the following Table 1.

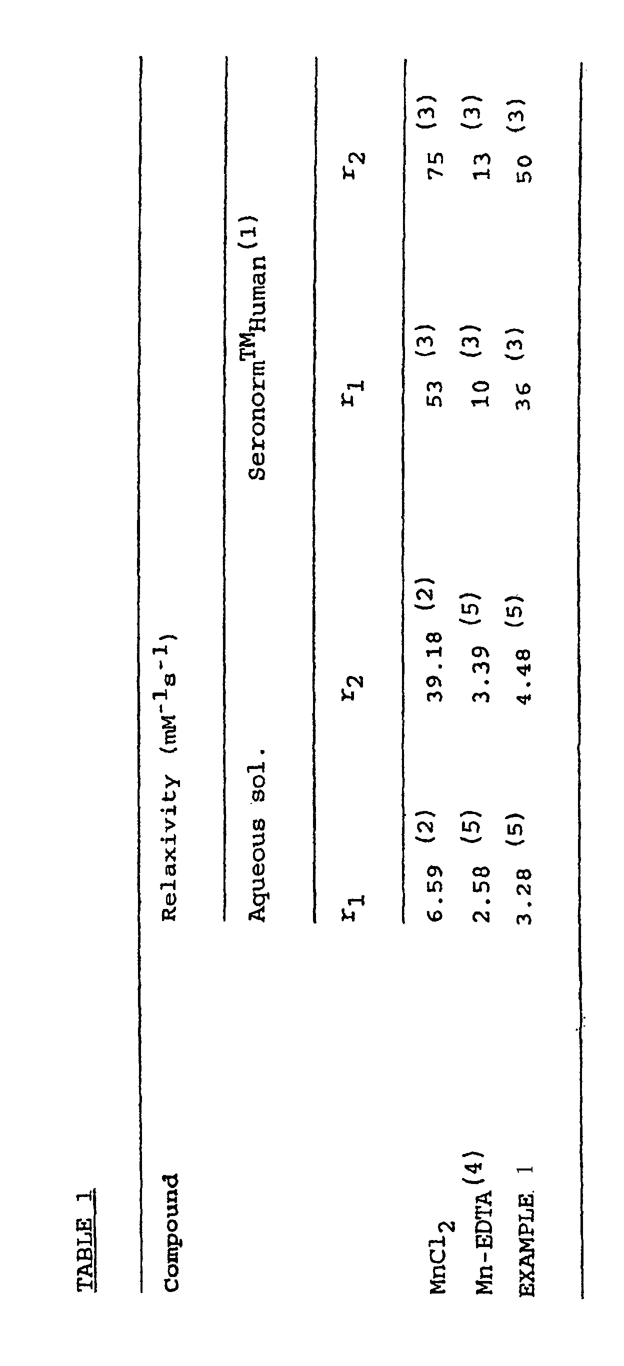

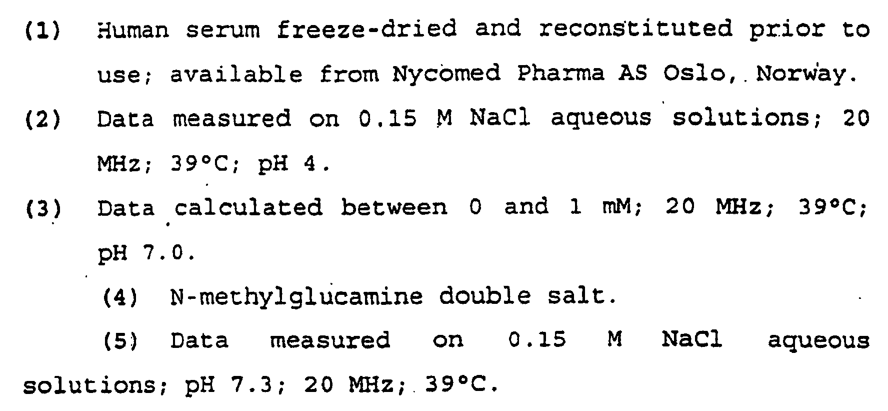

The high r₁ and, more specifically, r₂ relaxivity values measured in serum for the compounds of the invention, clearly show the remarkable potential of these compounds in M.R.I. diagnostic imaging, both for general use and, more particularly, for use in combinations of T₁/T₂ weighed or T₂ weighed sequences, as well as in the imaging of the cardiocirculatory system.

Following Table 2 shows the stability constants in aqueous solution relating to the compound of Example 1 , compared with Mn-EDTA.

**TABLE 2**

| **Compound** | **Log K**_{**ML**} ^{(**1**)} |
|---|---|
| Mn-EDTA | 13.88 ^{(**2**)} |
| EXAMPLE 1 | 13.8 ± 0.1 |

| | |
|---|---|
| (**1**) Thermodynamic stability constants, measured by the potentiometric method at 20°C in 0.1 M KCl aqueous solution. | |
| (**2**) Robert M. Smith and Arthur E. Martell: "Critical Stability Constants", Vol. 6, Second Supplement; Plenum Press, 1989. | |

Following Table 3 shows the DL₅₀ in the mouse after intravenous administration of the compound of Example 1, compared with the toxicity values reported in literature for MnCl₂ and Mn-EDTA.

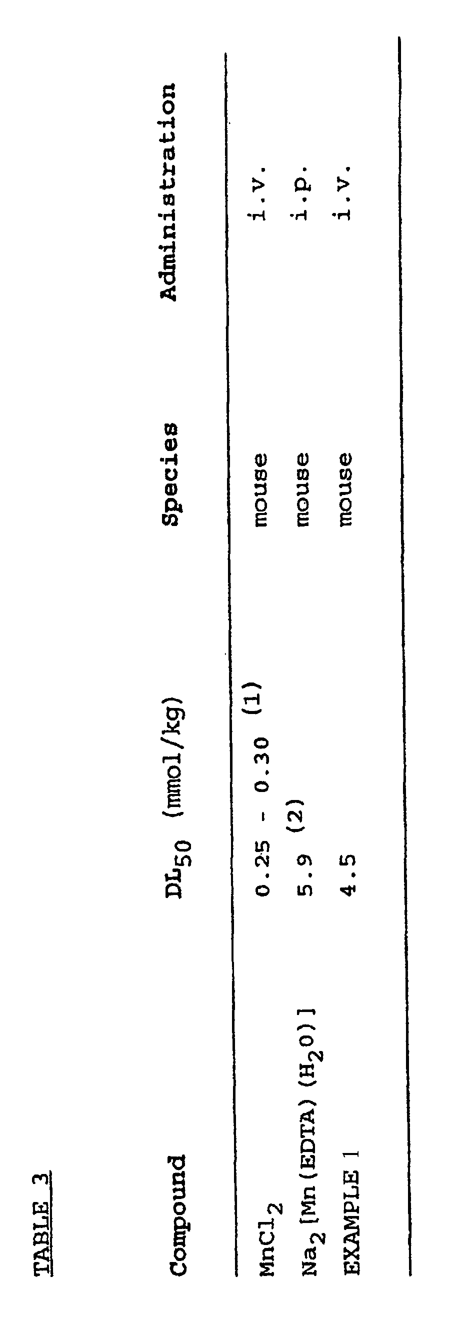

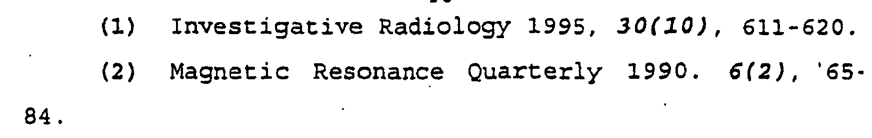

As it is shown by the data of this table, the compounds of the invention have quite favourable acute toxicity values.

## Claims

1. Compounds of formula (I): 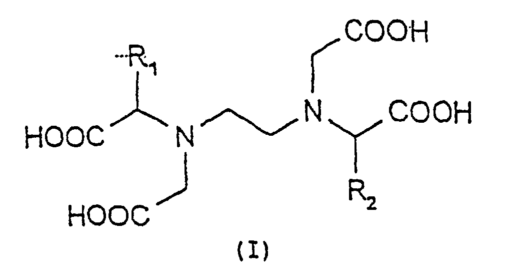 wherein:
R₁, R₂ which are the same or different, are a -(CH₂)ₘ-O-(CH₂)ₙ-R₃ group, wherein R₃ is an aryl residue, m is an integer 1 to 5 and n is an integer 1 to 4,
as well as the optically active forms of the chelates thereof with Mn(II) and the salts thereof with physiologically compatible organic bases selected from primary, secondary, tertiary amines or basic amino acids, or with inorganic bases whose cations are sodium, potassium, magnesium, calcium, or mixtures thereof.

2. Compounds as claimed in claim 1 salified with cations selected from:
- cations of inorganic bases, in particular, ions of alkali or alkaline-earth metals selected from sodium; potassium, magnesium, calcium, or mixtures thereof;
- cations of physiologically compatible organic bases selected from primary, secondary and tertiary amines, in particular, ethanolamine, diethanolamine, morpholine, glucamine, N-methylglucamine, N,N-dimethylglucamine;
- cations of amino acids, in particular lysine, arginine or ornithine.

3. Compounds as claimed in claim 2 salified with N-methylglucamine.

4. Compounds as claimed in any one of the above claims,
wherein R₁ and R₂ are residues of formulae: 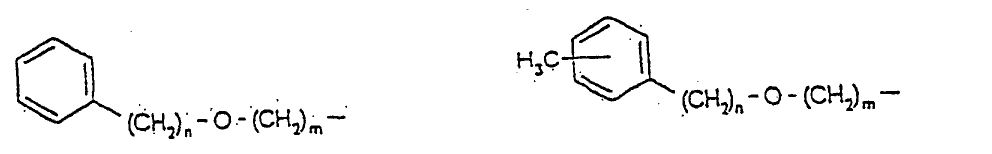 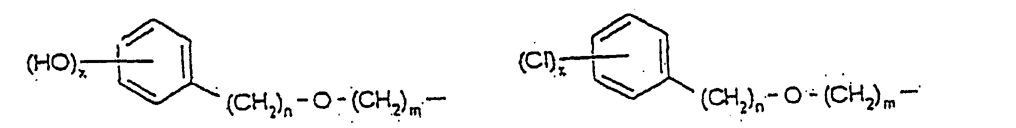 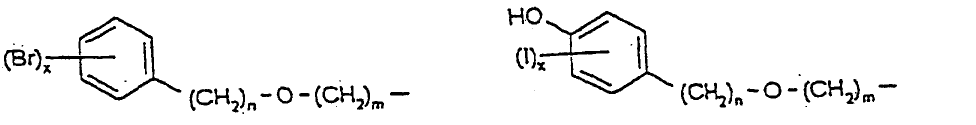 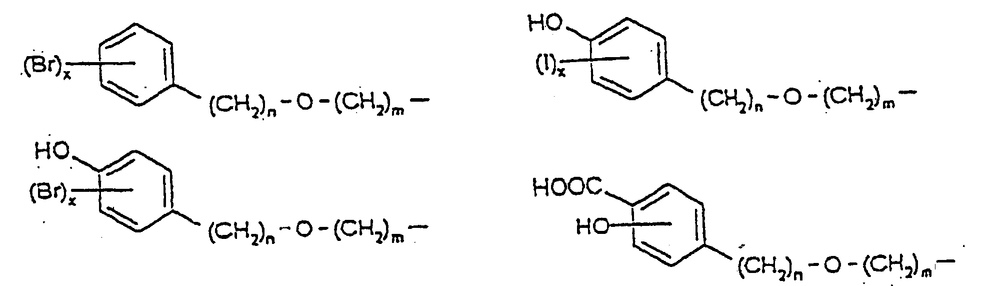 wherein: x = 1, 2.

5. Compounds as claimed in claim 1, of formula (II): 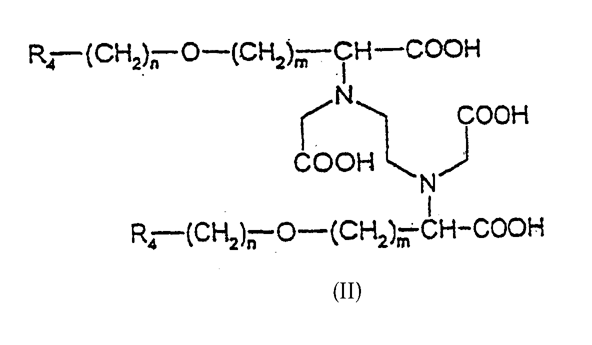 wherein R₄ is a phenyl, unsubstituted or substituted with one or more R₅ groups, which are the same or different, wherein R₅ is halogen, -OH, -NH₂, -COOH, -CONH₂, -SO₃H, -SO₃NH₂, or a C₁-C₄ alkyl chain.

6. Compounds as claimed in claim 5, in which R₄ is one of the residues of formulae: 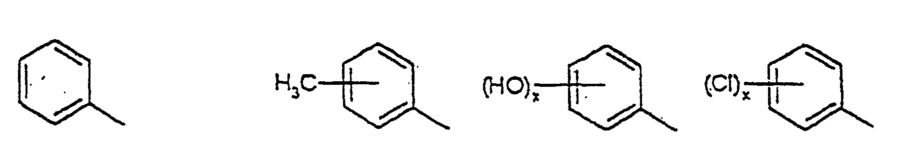 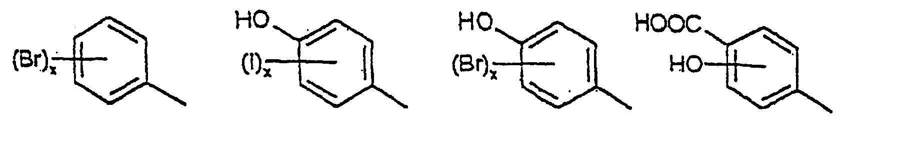 wherein: x = 1,2.

7. Compounds as claimed in claim 1, of formula (III) 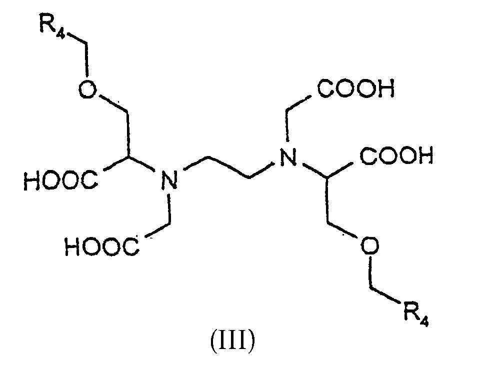 wherein the R₄ groups, which are the same or different, have same meanings as in claim 4.

8. Compounds as claimed in claim 7, in which R₄ is one of the following groups:

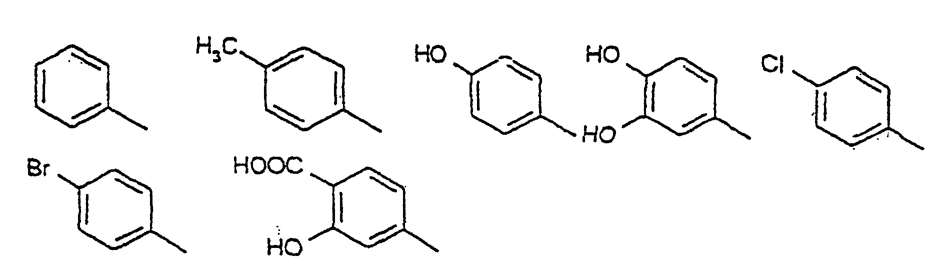

9. Compounds as claimed in claim 8, in which R₄ is unsubstituted phenyl.

10. Compound as claimed in claim 1, of formula:

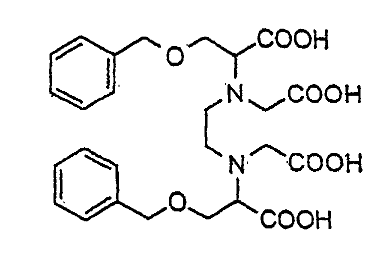

11. Contrastographic formulation for M.R.I. diagnostic imaging, comprising at least one of the chelated complexes according to claims 1 to 10, or a physiologically compatible salt thereof.

12. Compounds of claims 1 to 10, for M.R.I. diagnostic imaging of the liver and the biliary tract.

13. Compounds of claims 1 to 10, for M.R.I. diagnostic imaging of the cardiocirculatory system.

14. The use of chelated complexes according to any one of claims 1 to 10 for the preparation of M.R.I. diagnostic formulations wherein said formulations are used in the imaging of organs or tissues of the human or animal body.

15. The use of chelated complexes as claimed in claim 14 wherein the diagnostic formulations contain said chelated complexes in concentrations ranging from 0.001 to 1.0 mmol/ml.

16. The use of chelated complexes as claimed in claim 15 wherein the diagnostic formulations contain said chelated complexes in concentrations ranging from 0.01 to 0.5 mmol/ml.

17. The use of the chelated complexes as claimed in claim 15 wherein the formulations are administered to the patient in doses ranging from 0.001 to 0.1 mmol/kg.

## Patentansprüche

1. Verbindungen der Formel (I): 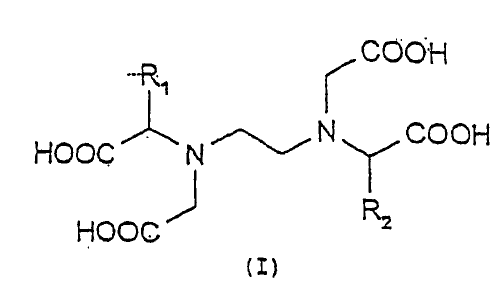 worin:
R₁ und R₂, die gleich oder verschieden sind, eine -(CH₂)ₘ-O-(CH₂)ₙ-R₃-Gruppe sind, worin R₃ für einen Arylrest steht, m eine ganze Zahl von 1 bis 5 ist und n eine ganze Zahl von 1 bis 4 ist,
sowie die optisch aktiven Formen der Chelate davon mit Mn(II) und die Salze davon mit physiologisch verträglichen organischen Basen, ausgewählt aus primären, sekundären und tertiären Aminen oder basischen Aminosäuren oder mit anorganischen Basen, deren Kationen Natrium, Kalium, Magnesium, Calcium oder Gemische davon sind.

2. Verbindungen nach Anspruch 1, **dadurch gekennzeichnet, dass** sie mit Kationen, ausgewählt aus:
- Kationen von anorganischen Basen, insbesondere Ionen von Alkali- oder Erdalkalimetallen, ausgewählt aus Natrium, Kalium, Magnesium, Calcium oder Gemischen davon;
- Kationen von physiologisch verträglichen organischen Basen, ausgewählt aus primären, sekundären und tertiären Aminen, insbesondere Ethanolamin, Diethanolamin, Morpholin, Glucamin, N-Methylglucamin, N,N-Dimethylglucamin;
- Kationen von Aminosäuren, insbesondere Lysin, Arginin oder Ornithin
ein Salz gebildet haben.

3. Verbindungen nach Anspruch 2, **dadurch gekennzeichnet, dass** sie mit N-Methylglucamin ein Salz gebildet haben.

4. Verbindungen nach einem der obigen Ansprüche, **dadurch gekennzeichnet, dass** R₁ und R₂ Reste der Formeln: 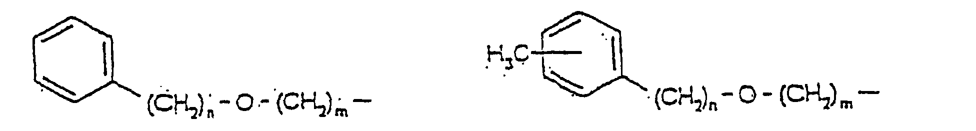 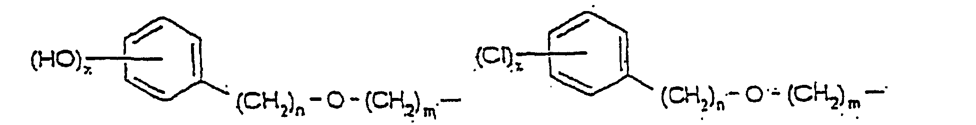 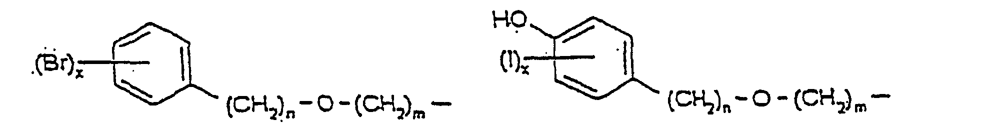 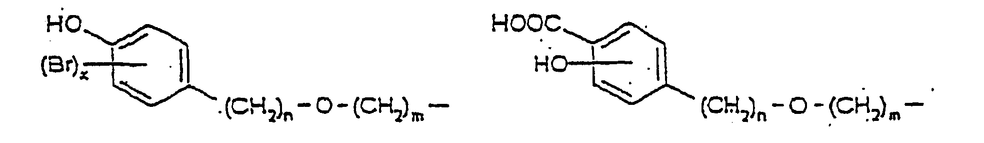 sind, worin: x = 1, 2.

5. Verbindungen nach Anspruch 1 der Formel (II): 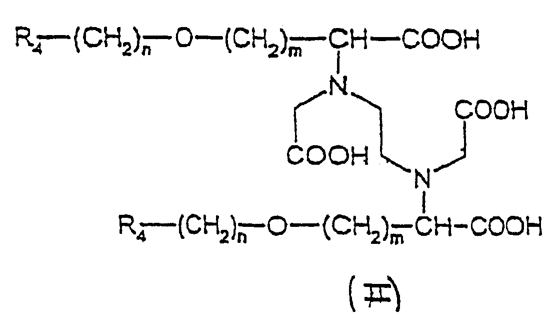 worin R₄ für Phenyl steht, das unsubstituiert ist oder mit einer oder mehreren Gruppen R₅, die gleich oder verschieden sind, substituiert ist, wobei R₅ für Halogen, -OH, -NH₂, -COOH, -CONH₂, -SO₃H, -SO₃NH₂ oder eine C₁-C₄-Alkylkette steht.

6. Verbindungen nach Anspruch 5, **dadurch gekennzeichnet, dass** R₄ einer der Reste der Formeln: 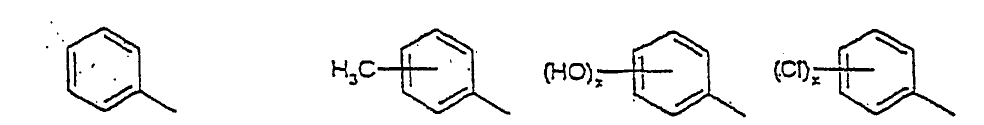 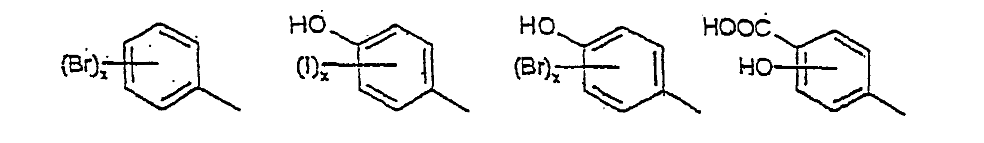 ist, worin: x = 1, 2.

7. Verbindungen nach Anspruch 1 der Formel (III), 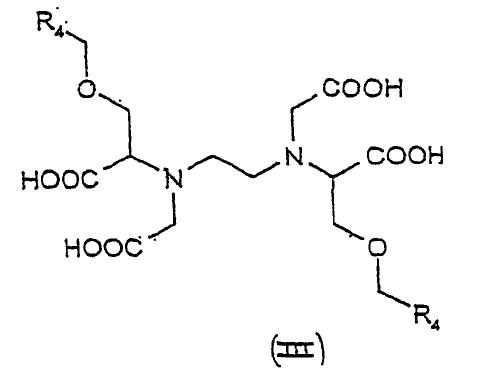 worin die Gruppen R₄, die gleich oder verschieden sind, die gleichen Bedeutungen wie in Anspruch 4 haben.

8. Verbindungen nach Anspruch 7, **dadurch gekennzeichnet, dass** R₄ eine der folgenden Gruppen ist:

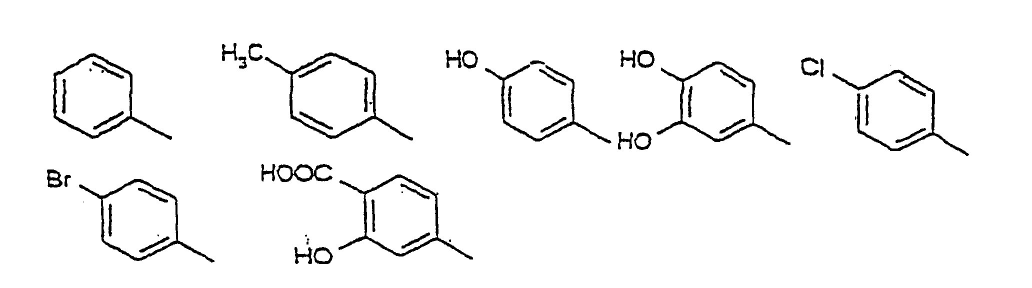

9. Verbindungen nach Anspruch 8, **dadurch gekennzeichnet, dass** R₄ unsubstituiertes Phenyl ist.

10. Verbindung nach Anspruch 1 der Formel:

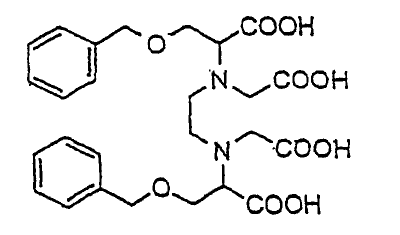

11. Kontrastographische Zubereitung für die diagnostische M.R.I.-Abbildung, umfassend mindestens einen der chelatisierten Komplexe nach den Ansprüchen 1 bis 10, oder ein physiologisch verträgliches Salz davon.

12. Verbindungen nach den Ansprüchen 1 bis 10 für die diagnostische M.R.I.-Abbildung der Leber und des Gallentrakts.

13. Verbindungen nach den Ansprüchen 1 bis 10 für die diagnostische M.R.I.-Abbildung des kardiovaskulären Systems.

14. Verwendung der chelatisierten Komplexe nach einem der Ansprüche 1 bis 10 zur Herstellung von diagnostischen M.R.I.-Zubereitungen, wobei die genannten Zubereitungen für die Abbildung von Organen oder Geweben des menschlichen oder tierischen Körpers verwendet werden.

15. Verwendung der chelatisierten Komplexe nach Anspruch 14, **dadurch gekennzeichnet, dass** die diagnostischen Zubereitungen die genannten chelatisierten Komplexe in Konzentrationen von 0,001 bis 1,0 mmol/ml enthalten.

16. Verwendung von chelatisierten Komplexen nach Anspruch 15, **dadurch gekennzeichnet**, die diagnostischen Zubereitungen die genannten chelatisierten Komplexe in Konzentrationen von 0,01 bis 0,5 mmol/ml enthalten.

17. Verwendung von chelatisierten Komplexen nach Anspruch 15, **dadurch gekennzeichnet, dass** die Zubereitungen dem Patienten in Dosen in Bereichen von 0,001 bis 0,1 mmol/kg verabreicht werden.

## Revendications

1. Composés de formule (I) : 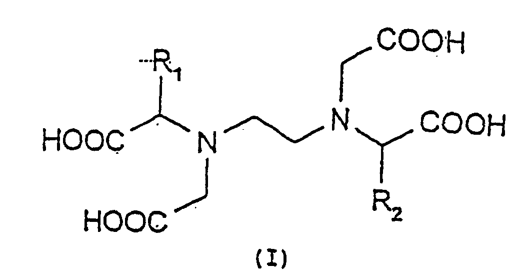 dans laquelle :
R₁, R₂, qui sont identiques ou différents, sont chacun un groupe -(CH₂)ₘ-O-(CH₂)ₙ-R₃ où R₃ est un résidu aryle, m est un nombre entier de 1 à 5 et n est un nombre entier de 1 à 4,
ainsi que les formes optiquement actives de leurs chélates formés avec Mn(II) et leurs sels formés avec des bases organiques physiologiquement compatibles choisies parmi les amines primaires, secondaires, tertiaires ou les acides aminés basiques, ou avec des bases minérales dont les cations sont le sodium, le potassium, le magnésium, le calcium, ou leurs mélanges.

2. Composés tels que revendiqués dans la revendication 1, salifiés avec des cations choisis parmi :
- les cations de bases minérales, en particulier des ions de métaux alcalins ou alcalino-terreux choisis parmi le sodium, le potassium, le magnésium, le calcium ou des mélanges d'entre eux ;
- les cations de bases organiques physiologiquement compatibles choisies parmi les amines primaires, secondaires et tertiaires, en particulier l'éthanolamine, la diéthanolamine, la morpholine, la glucamine, la N-méthylglucamine, la N,N-diméthylglucamine ;
- les cations d'acides aminés, en particulier la lysine, l'arginine ou l'ornithine.

3. Composés tels que revendiqués dans la revendication 2, salifiés avec la N-méthylglucamine.

4. Composés tels que revendiqués dans l'une quelconque des revendications ci-dessus, dans lesquels R₁ et R₂ sont des résidus de formules : 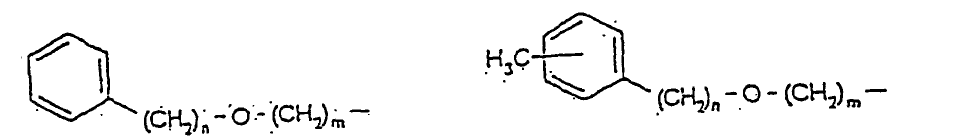 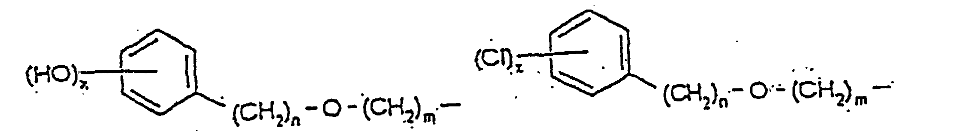 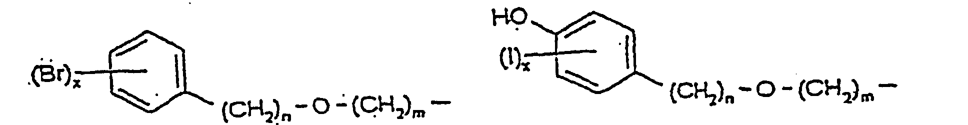 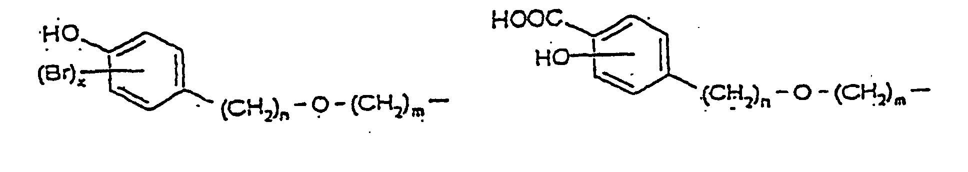 où : x = 1, 2.

5. Composés tels que revendiqués dans la revendication 1, de formule (II) : 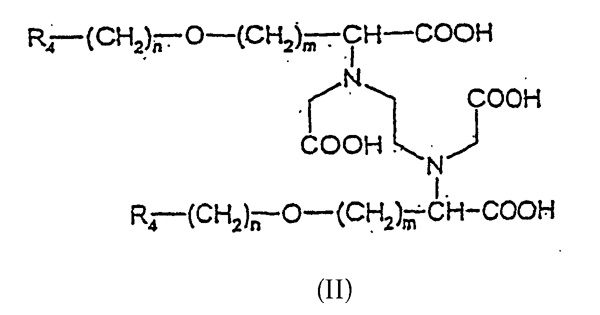 où R₄ est un groupe phényle, non substitué ou substitué avec un ou plusieurs groupes R₅, qui sont identiques ou différents, R₅ étant un halogène, -OH, -NH₂, -COOH, -CONH₂, -SO₃H, -SO₃NH₂ ou une chaîne alkyle en C₁-C₄.

6. Composés tels que revendiqués dans la revendication 5, dans lesquels R₄ est l'un des résidus de formules : 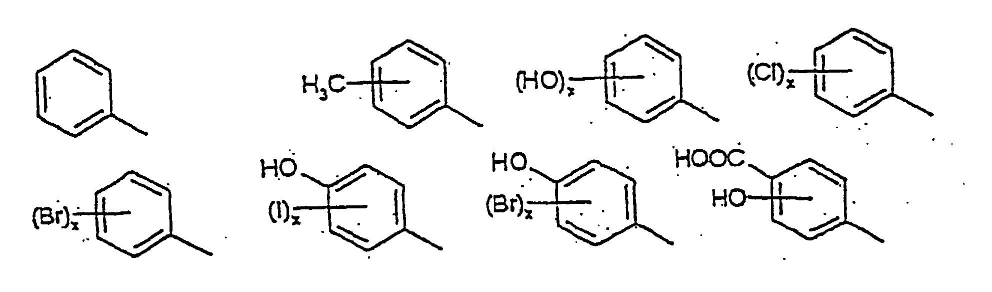 où : x = 1, 2.

7. Composés tels que revendiqués dans la revendication 1, de formule (III) 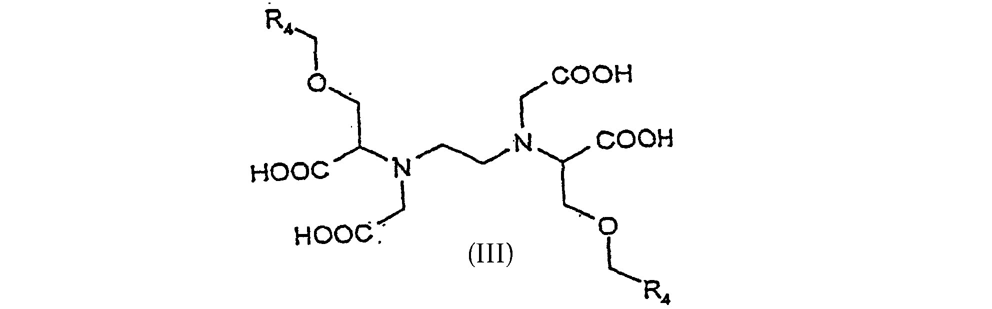 où les groupes R₄, qui sont identiques ou différents, ont les mêmes significations que dans la revendication 4.

8. Composés tels que revendiqués dans la revendication 7, dans lesquels R₄ est l'un des groupes suivants

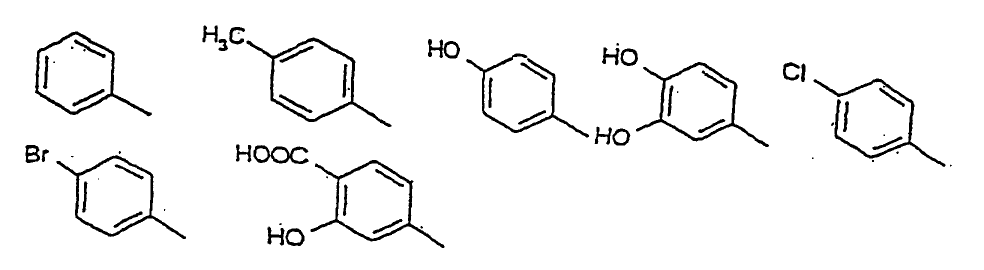

9. Composés tels que revendiqués dans la revendication 8, dans lesquels R₄ est un groupe phényle non substitué.

10. Composé tel que revendiqué dans la revendication 1, de formule :

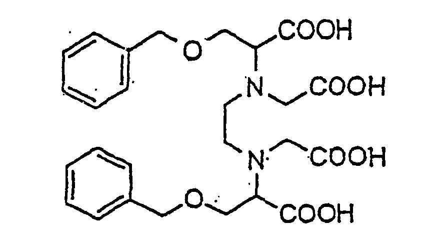

11. Formulation contrastographique pour imagerie diagnostique par I.R.M., comprenant au moins l'un des complexes chélatés selon les revendications 1 à 10, ou un sel de celui-ci physiologiquement compatible.

12. Composés des revendications 1 à 10, pour imagerie diagnostique par I.R.M. du foie et des voies biliaires.

13. Composés des revendications 1 à 10, pour imagerie diagnostique par I.R.M. du système cardiocirculatoire.

14. L'utilisation de complexes chélatés selon l'une quelconque des revendications 1 à 10, pour la préparation de formulations diagnostiques pour I.R.M., dans laquelle lesdites formulations sont utilisées dans la visualisation d'organes ou de tissus du corps humain ou animal.

15. L'utilisation de complexes chélatés telle que revendiquée dans la revendication 14, dans laquelle les formulations diagnostiques contiennent lesdits complexes chélatés à des concentrations comprises entre 0,001 et 1,0 mmol/ml.

16. L'utilisation de complexes chélatés telle que revendiquée dans la revendication 15, dans laquelle les formulations diagnostiques contiennent lesdits complexes chélatés à des concentrations comprises entre 0,01 et 0,5 mmol/ml.

17. L'utilisation des complexes chélatés telle que revendiquée dans la revendication 15, dans laquelle les formulations sont administrées au patient à des doses comprises entre 0,001 et 0,1 mmol/kg.
